# EUROPÄISCHE PATENTSCHRIFT

(11) **EP 0 908 438 B1**
(45) Veröffentlichungstag und Bekanntmachung des Hinweises auf die Patenterteilung: **26.11.2003**
(21) Anmeldenummer: 98116713.3
(22) Anmeldetag: 03.09.1998
(51) Int. Cl.: C07C 17/20, C07C 25/13, C07C 51/62, C07C 253/30

(54) **Verfahren zur Herstellung von 2,3,4,5-Tetrafluorbenzolderivaten**
Process for the preparation of 2,3,4,5-tetrafluorobenzene derivatives
Procédé pour la préparation de dérivés de 2,3,4,5-tétrafluorobenzène

(30) Priorität: 16.09.1997 DE 19740632
(43) Veröffentlichungstag der Anmeldung: 14.04.1999
(73) Patentinhaber: BAYER AG, 51368 Leverkusen (DE)
(72) Erfinder: Neuner, Otto, Dr., 51465 Bergisch Gladbach (DE); Lui, Norbert, Dr., 51061 Köln (DE); Bielefeldt, Dietmar, Dr., 40883 Ratingen (DE); Holzbrecher, Michael, 51766 Engelskirchen (DE)

(56) Entgegenhaltungen:
- EP-A- 0 607 824
- DE-A- 3 420 796
- DATABASE CHEMABS [Online] CHEMICAL ABSTRACTS SERVICE, COLUMBUS, OHIO, US; STN; CASREACT accession no. 117:69574, XP002165317 -& CHEMICAL ABSTRACTS, vol. 117, no. 7, 17. August 1992 (1992-08-17) Columbus, Ohio, US; abstract no. 69574, XP002165316 & JP 04 049263 A (ASAHI GARASU KK)

## Beschreibung

Die vorliegende Erfindung betrifft ein verbessertes Verfahren zur Herstellung von 2,3,4,5-Tetrafluorbenzolderivaten aus 2,3,4,5-Tetrachlor- und/oder 2,3,4,5-Tetra-(chlor, fluor)-benzolderivaten durch Chlor/Fluor-Austausch.

In der DE-A 3 420 796 ist ein Verfahren zur Herstellung von 2,3,4,5-Tetrafluorbenzolderivaten beschrieben, bei dem man 2,3,4,5-Tetrachlorbenzolderivate mit Kaliumfluorid in einem Lösungsmittel bei erhöhter Temperatur umsetzt. Geht man dabei von 2,3,4,5-Tetrachlorbenzoylfluorid aus und destilliert das Gemisch der Fluorierungsprodukte bei 800 bis 500 mbar kontinuierlich ab, so erhält man nach fraktionierter Destillation des Gemisches der Fluorierungsprodukte das gewünschte 2,3,4,5-Tetrafluorbenzoylfluorid in einer Ausbeute von lediglich 10 % d.Th., während wesentlich mehr unerwünschte Fluorierungsprodukte erhalten werden, nämlich 2,3,4-Trifluor-5-chlorbenzoylfluorid in einer Ausbeute von 43 % d.Th. und 2,4-Difluor-3,5-dichlorbenzoylfluorid in einer Ausbeute von 14 % d.Th. (s. Beispiel 2 der DE-A). Wenn man von 2,3,4,5-Tetrachlorbenzonitril ausgeht und den Fluorierungsansatz fraktioniert destilliert sind die Verhältnisse offenbar ähnlich, allerdings werden keine Mengenangaben zu den erhaltenen Tetrafluor-, Trifluorchlor- und Difluordichlorbenzonitrilen gemacht. (s. Beispiel 5 der DE-A).

Die EP-A 607 824 beschreibt ein Verfahren zur Herstellung von 2,3,4,5-Tetrafluorbenzotrifluorid durch Umsetzung von Tetrachlorbenzotrifluorid mit Kaliumfluorid. Wenn man dabei von reinem 2,3,4,5-Tetrachlorbenzotrifluorid ausgeht und das Reaktionsgemisch destillativ aufarbeitet erhält man ein Rohdestillat, das 2,3,4,5-Tetrafluorbenzotrifluorid in einer Menge von 54 bis 62 % d.Th. enthält (s. Beispielgruppe A. 4. Beispiel, Teile a), b), c) und e) der EP-A). Wenn man von Gemischen aus 2,3,4,5-Tetrachlor- und 2,3,4-Trifluor-5-chlorbenzotrifluorid ausgeht, sinkt der Gehalt des Rohdestillats an 2,3,4,5-Tetrafluorbenzotrifluorid auf 51 % d.Th. (s. Beispiel A, 4. Beispiel, Teil d) der EP-A). Bei Wiederfindungsraten (d.h. der Summe der Ausbeuten an 2,3,4,5-Tetrafluor- und 2,3,4-Trifluor-5-chlorbenzotrifluorid) von ca. 90 % liegen die Ausbeuten an 2,3,4,5-Tetrafluorbenzotrifluorid bei nur 50 bis 55 % d.Th., die Ausbeuten an 2,3,4-Trifluor-5-chlor-benzotrifluorid jedoch bei 34 bis 38 % d.Th. (s. Beispielgruppe A, 4. Beispiel, Teile d) und e) der EP-A). Das bedeutet, daß zur Herstellung von 2,3,4,5-Tetrafluorbenzotrifluorid nahezu 40 bis 50 % der Wiederfindungsrate an 2,3,4-Trifluor-5-chlor-benzotrifluorid recyclisiert werden muß und damit der Aufwand hoch und die Raum-Zeit-Ausbeute niedrig ist.

Es besteht deshalb noch immer ein Bedürfnis nach einem Verfahren zur Herstellung von 2,3,4,5-Tetrafluorbenzolderivaten, bei dem man die gewünschten Produkte in guten Ausbeuten und Selektivitäten erhält.

Es wurde nun ein Verfahren zur Herstellung von Tetrafluorbenzolderivaten der Formel (I) gefunden 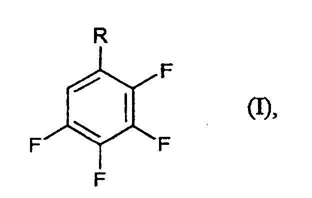 in der
- R: für CF₃, CN oder COF steht,
bei dem man ein Chlorbenzolderivat der Formel 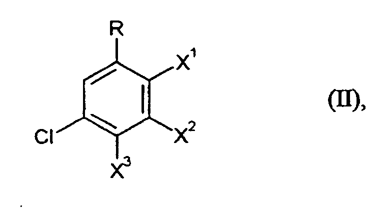 in der
- R: die bei Formel (I) angegebene Bedeutung hat und
- X¹, X² und X³: unabhängig voneinander jeweils für F oder Cl stehen,
mit einem Fluorierungsmittel in Gegenwart eines Lösungsmittels und eines Katalysators bei erhöhter Temperatur umsetzt, das dadurch gekennzeichnet ist, daß man die Reaktion zunächst bei solchen Temperatur- und Druckbedingungen durchführt, daß die jeweilige Verbindung der Formel (I) kontinuierlich durch eine Kolonne abdestilliert und anschließend solche Temperatur- und Druckbedingungen einstellt, daß restliche Anteile der Verbindung der Formel (I) zusammen mit der entsprechenden 2,3,4-Trifluor-5-chlor-Verbindung abdestillieren.

In das erfindungsgemäße Verfahren werden vorzugsweise Verbindungen der Formel (II) eingesetzt, bei denen X¹, X² und X³ für Chlor stehen, oder solche Verbindungen der Formel (II) im Gemisch mit Verbindungen der Formel (II), bei denen X¹, X² und X³ für Fluor stehen.

Als Fluorierungsmittel kommen z.B. Alkalimetallfluoride in Frage, wie Natriumfluorid, Kaliumfluorid, Cäsiumfluorid und Gemische der genannten Fluoride. Bevorzugt ist Kaliumfluorid, insbesondere in sprühgetrockneter Form.

Pro Äquivalent auszutauschender Chloratome kann man z.B. mindestens 1 Äquivalent Fluorierungsmittel einsetzen. Vorzugsweise liegt diese Menge bei 1,1 bis 1,5 Äquivalenten Fluorierungsmittel. Größere Mengen sind nicht kritisch, jedoch nicht ökonomisch.

Als Lösungsmittel kommen dipolare aprotische Lösungsmittel in Frage, wie Tetramethylensulfon (Sulfolan), N-Methylpyrrolidon, Dimethylsulfoxid, Dimethylformamid, Dimethylacetamid, Diglyme, Tetraglyme und 1,3-Dimethylimidazolidinon. Bevorzugt ist Tetramethylensulfon (Sulfolan). Das Lösungsmittel kann man beispielsweise in einer Menge von 1 kg bis 10 kg, bezogen auf 1 kg Fluorierungsreagenz, einsetzen. Größere Mengen sind nicht kritisch, jedoch nicht ökonomisch.

Als Katalysatoren kommen z.B. sog. Phasentransferkatalysatoren in Frage wie quarternäre Ammoniumsalze, Pyridiniumsalze, quartemäre Phosphoniumsalze und Kronenether. Als Einzelbeispiele seien genannt: Tetramethylammoniumchlorid, Tetrabutylammoniumbromid, N-Neopentyl-4-(N',N'-dimethylamino)-pyridiniumchlorid, Tetrabutylphosphoniumbromid, Tetraphenylphosphoniumbromid und 18-Krone-6. Bevorzugt ist Tetraphenylphosphoniumbromid. Bezogen auf die Ausgangsverbindung(en) der Formel (II) kann man z.B. 1 bis 50 Gew.-%, vorzugsweise 4 bis 15 Gew.-% Katalysator, bezogen auf das Einsatzmaterial, einsetzen.

Die Reaktionstemperatur kann z.B. im Bereich 160 bis 250°C liegen. Vorzugsweise liegt sie im Bereich 190 bis 230°C.

Es ist ein wesentliches Merkmal der vorliegenden Erfindung, daß man bei gegebener Reaktionstemperatur den Druck zunächst so einstellt, daß die jeweilige Verbindung der Formel (I) kontinuierlich abdestilliert. Welcher Druck hier im Einzelfall optimal ist, kann durch routinemäßige Vorversuche einfach ermittelt werden. Es hat sich gezeigt, daß man hierfür i.a. Drucke im Bereich von z.B. 1,3 bis 6 bar, häufig im Bereich von 2 bis 4 bar benötigt.

Die während der Reaktion entstehende Verbindung der Formel (I) ist i.a. die am tiefsten siedende Komponente des Reaktionsgemisches. Der Druck wird zunächst so gewählt, daß praktisch nur die entstehende Verbindung der Formel (I) abdestilliert und praktisch keine höher siedenden Bestandteile des Reaktionsgemisches. Im Falle des Einsatzes von Tetrachlorbenzotrifluorid und einer Reaktionstemperatur von 200 bis 220°C haben sich beispielsweise Drucke im Bereich 2,1 bis 2,3 bar bewährt.

Nach dem Ende der Umsetzung geht praktisch keine Verbindung der Formel (I) mehr über und der Druck sinkt. Ab diesem Zeitpunkt werden erfindungsgemäß solche Druck- und Temperaturbedingungen eingestellt, daß im Reaktionsgemisch noch vorhandene Anteile der Verbindung der Formel (I) zusammen mit der entsprechenden 2,3,4-Trifluor-5-chlor-Verbindung abdestillieren. Hierzu reicht es i.a. aus, lediglich den Druck abzusenken, z.B. bis in einem Bereich von 50 bis 200 mbar. Es ist bevorzugt, die erste Fraktion (= praktisch reine Verbindung der Formel (I)) und die zweite Fraktion (= Gemisch der Verbindung der Formel (I) und der entsprechenden 2,3,4-Trifluor-5-chlor-Verbindung) getrennt voneinander aufzufangen.

Die zweite Fraktion setzt man ganz, oder in vorteilhafter Weise nur die darin enthaltenen teilfluorierten Verbindungen nach deren Abtrennung, dem Folge-Ansatz zu, um die erfindungsgemäße Umsetzung zur Verbindung der Formel (I) durchzuführen.

Auf die erfindungsgemäße Weise erhält man Verbindungen der Formel (I) in deutlich besseren Ausbeute und Selektivitäten als bisher. Beim erfindungsgemäßen Verfahren liegen die Ausbeuten an Verbindungen der Formel (I) beispielsweise im Bereich von 65 bis 75 % d.Th. und die Ausbeuten an entsprechenden 2,3,4-Trifluor-5-chlor-Verbindungen beispielsweise im Bereich von nur 12 bis 18 % d.Th. Das bedeutet, im geraden Durchgang wird beim erfindungsgemäßen Verfahren deutlich mehr Produkt der Formel (I) und deutlich weniger ensprechende 2,3,4-Trifluor-5-chlor-Verbindung erhalten als gemäß der EP-A-607 824. Das hat insbesondere zur Folge, daß erfindungsgemäß nur etwa halb so viel 2,3,4-Trifluor-5-chlor-Verbindungen recyclisiert werden müssen. Damit können die gewünschten Verbindungen der Formel (I) mit wesentlich weniger Aufwand (z.B. an Lösungsmittel, Fluorierungsmitteln, Katalysatoren, Destillationsaufwand etc.) und mit wesentlich höheren Raum-Zeit-Ausbeuten hergestellt werden als bisher.

### Beispiele

### Beispiel 1

In einem 5 l Autoklaven aus Edelstahl mit Rührer, Kolonne, Rücklaufteiler, Kühler mit Druckhalteventil und 1 000 ml Wechselvorlage wurde ein durch Andestillieren vorgetrocknetes Gemisch aus 2 750 g Sulfolan (rein) und 1 000 g Kaliumfluorid (wasserfrei) vorgelegt, 50 g Tetraphenylphosphoniumbromid und 1 050 g 2,3,4,5-Tetrachlorbenzotrifluorid (destilliert) eingetragen und bei druckdicht verschlossenem Autoklaven das Gemisch unter. Rühren auf 210°C geheizt. Durch die einsetzende Bildung von 2,3,4,5-Tetrafluorbenzotrifluorid und 2,3,4-Trifluor-5-chlorbenzotrifluorid stieg der Gesamtdruck in der Anlage allmählich bis auf 2,2 bar an. Sobald in der Kolonne unter den Reaktionsbedingungen (Gesamtdruck: 2,2 bar und Innentemperatur: 210°C) die Kopftemperatur auf 130°C anstiegen war, wurde mit einem Rücklaufverhältnis von A:R = 1:10 Destillat in die Vorlage abgenommen. Während der Destillatabnahme wurden die Reaktions- und Destillationsbedingungen konstant gehalten. Gegen Ende der Umsetzung fiel der Gesamtdruck ab und die Destillation des 2,3,4,5-Tetrafluorbenzotrifluorid ließ merklich nach. Nach dem Ende der Umsetzung und Abfallen des Gesamtdrucks wurde durch Entspannen bis auf einen Druck von 1 bar ein Gemisch aus 2,3,4,5-Tetrafluor- und 2,3,4-Trifluor-5-chlorbenzotrifluorid abgenommen. Durch anschließendes Einstellen eines Druckes von 50 bis 100 mbar wurden dann die in Sulfolan noch vorhandenen flüchtigen Anteile bis zum Erreichen des Siedepunktes von Sulfolan in die entleerte Vorlage destilliert. In einer Gesamtausbeute von 85 % d.Th., bezogen auf eingesetztes 2,3,4,5-Tetrachlorbenzo-trifluorid wurden 565 g (= 70 % d.Th.) 2,3,4,5-Tetrafluorbenzotrifluorid und 130 g (15 % d.Th.) 2,3,4-Trifluor-5-chlor-benzotrifluorid erhalten. Das teilfluorierte Benzotrifluorid wurde dem Folgeansatz zur Herstellung von 2,3,4,5-Tetrafluorbenzotrifluorid zugesetzt.

### Beispiel 2

In einem 5 l Autoklaven wurde ein Gemisch aus 2 700 g Sulfolan (rein) und 980 g Kaliumfluorid (wasserfrei) vorgelegt, 55 g Tetraphenylphosphoniumbromid und ein Gemisch aus 1 000 g 2,3,4,5-Tetrachlorbenzotrifluorid und 130 g 2,3,4-Trifluor-5-benzotrifluorid zugefügt und im übrigen verfahren wie in Beispiel 1 beschrieben. Nach dem fraktionierten Abdestillieren der gesamten flüchtigen Anteile wurden in einer Ausbeute von 86 % d.Th., bezogen auf eingesetztes 2,3,4,5-Tetrachlorbenzotrifluorid und 2,3,4-Trifluor-5-chlorbenzotrifluorid, 635 g (= 71,5 % d.Th.) 2,3,4,5-Tetrafluorbenzotrifluorid und 140 g (= 14,6 % d.Th.) 2,3,4-Trifluor-5-chlorbenzotrifluorid erhalten.

### Beispiel 3

In einem 130 l Autoklaven aus 1.4571 mit Blattrührer, Kolonne, Rücklaufteiler, Kühler mit Druckhalteventil und Wechselvorlage, wurden 61 kg Sulfolan, rein und 20 kg Kaliumfluorid, wasserfrei vorgelegt und das Gemisch durch Abdestillieren von 10 kg Sulfolan entwässert. Zu der ca. 180°C warmen Suspension wurden danach zunächst die Lösung von 1 kg Tetraphenylphosphoniumbromid in 6 kg Sulfolan, wasserfrei und, nach weiterem Erwärmen auf 210°C, bei druckdicht verschlossenem Autoklaven, 19,5 kg 2,3,4,5-Tetrachlorbenzotrifluorid, dest. eingepumpt.

Unter weiterem Rühren bei 210°C bis 220°C stieg der Druck allmählich bis auf ca. 2,2 bar an.

Die Weiterverarbeitung des Ansatzgemisches erfolgte wie im Beispiel 1 beschrieben.

Nach Aufarbeitung des Ansatzes wurden in einer Gesamtausbeute von 91,6% bez. auf 2,3,4,5-Tetrachlorbenzotrifluorid, dest. 11,2 kg 2,3,4,5-Tetrafluorbenzotrifluorid (= 74,8 % d.Th.) und 2,7 kg 2,3,4-Tri-fluor-5-chlorbenzotrifluorid (= 16,8 % d.Th.) erhalten.

## Patentansprüche

1. Verfahren zur Herstellung von Tetrafluorbenzolderivaten der Formel (I) 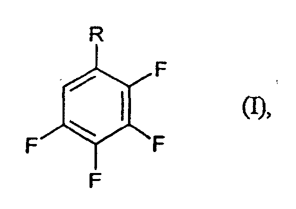 in der
R für CF₃, CN oder COF steht,
bei dem man ein Chlorbenzolderivat der Formel 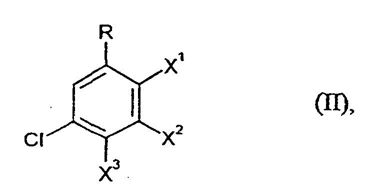 in der
R die bei Formel (I) angegebene Bedeutung hat und
X¹, X² und X³ unabhängig voneinander jeweils für F oder Cl stehen,
mit einem Fluorierungsmittel in Gegenwart eines Lösungsmittels und eines Katalysators bei erhöhter Temperatur umsetzt, **dadurch gekennzeichnet, daß** man die Reaktion zunächst bei solchen Temperatur- und Druckbedingungen durchführt, daß die jeweilige Verbindung der Formel (I) kontinuierlich durch eine Kolonne abdestilliert und anschließend solche Temperatur- und Druckbedingungen einstellt, daß restliche Anteile der Verbindung der Formel (I) zusammen mit der entsprechenden 2,3,4-Trifluor-5-chlor-Verbindung abdestillieren.

2. Verfahren nach Anspruch 1, **dadurch gekennzeichnet, daß** die Reaktionstemperatur im Bereich 160 bis 250°C liegt.

3. Verfahren nach Ansprüchen 1 und 2, **dadurch gekennzeichnet, daß** man bei gegebener Reaktionstemperatur den Druck zunächst so einstellt, daß die jeweilige Verbindung der Formel (I) kontinuierlich abdestilliert.

4. Verfahren nach Anspruch 3, **dadurch gekennzeichnet, daß** man den Druck zunächst auf 1,3 bis 6 bar einstellt.

5. Verfahren nach Ansprüchen 1 bis 4, **dadurch gekennzeichnet, daß** man dann, wenn praktisch keine Verbindung der Formel (I) mehr über geht, den Druck auf 50 bis 200 mbar absenkt.

6. Verfahren nach Ansprüchen 1 bis 5, **dadurch gekennzeichnet, daß** in Formel (II) X¹, X² und X³ für Chlor stehen.

7. Verfahren nach Ansprüchen 1 bis 6, **dadurch gekennzeichnet, daß** man Gemische von Verbindungen der Formel (II), bei denen X¹, X² und X³ für Chlor stehen, mit Verbindungen der Formel (II), bei denen X¹, X² und X³ für Fluor stehen, einsetzt.

8. Verfahren nach Ansprüchen 1 bis 7, **dadurch gekennzeichnet, daß** man als Fluorierungsmittel Alkalimetallfluoride einsetzt.

9. Verfahren nach Ansprüchen 1 bis 8, **dadurch gekennzeichnet, daß** man als Lösungsmittel dipolare aprotische Lösungsmittel einsetzt.

10. Verfahren nach Ansprüchen 1 bis 9, **dadurch gekennzeichnet, daß** man als Katalysatoren Phasentransferkatalysatoren einsetzt.

## Claims

1. Process for the preparation of tetrafluorobenzene derivatives of the formula (I) 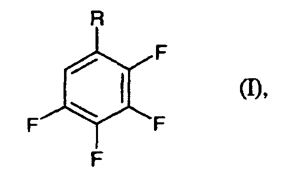 in which
R is CF₃, CN or COF,
by reacting a chlorobenzene derivative of the formula 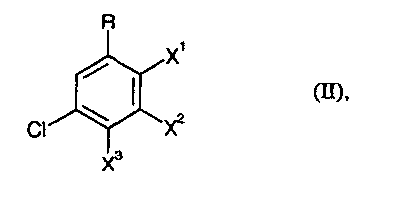 in which
R is as defined for formula (I), and
X¹, X² and X³ independently of one another are each F or Cl,
with a fluorinating agent in the presence of a solvent and a catalyst at elevated temperature, **characterized in that** it comprises carrying out the reaction initially under temperature and pressure conditions such that the respective compound of the formula (I) is continuously distilled off through a column, and subsequently setting the temperature and pressure conditions such that remaining fractions of the compound of the formula (I) are distilled off together with the corresponding 2,3,4-trifluoro-5-chloro compound.

2. Process according to Claim 1, **characterized in that** the reaction temperature is in the range from 160 to 250°C.

3. Process according to Claims 1 and 2, **characterized in that**, for a given reaction temperature, the pressure is initially set such that the respective compound of the formula (I) is continuously distilled off.

4. Process according to Claim 3, **characterized in that** the pressure is initially set at from 1.3 to 6 bar.

5. Process according to Claims 1 to 4, **characterized in that**, when virtually no more of compound of the formula (I) passes over, the pressure is reduced to from 50 to 200 mbar.

6. Process according to Claims 1 to 5, **characterized in that**, in formula (II) X¹, X² and X³ are chlorine.

7. Process according to Claims 1 to 6, **characterized in that** mixtures of compounds of the formula (II) in which X¹, X² and X³ are chlorine, with compounds of the formula (II) in which X¹, X² and X³ are fluorine are used.

8. Process according to Claims 1 to 7, **characterized in that** the fluorinating agent used is an alkali metal fluoride.

9. Process according to Claims 1 to 8, **characterized in that** the solvent used is a dipolar aprotic solvent.

10. Process according to Claims 1 to 9, **characterized in that** the catalyst used is a phase transfer catalyst.

## Revendications

1. Procédé pour la préparation de dérivés du tétrafluorobenzène répondant à la formule (I) 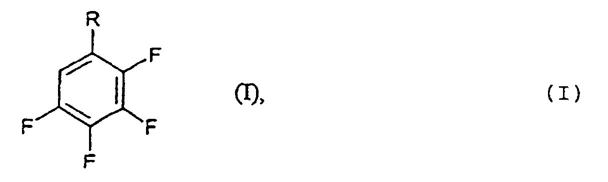 dans laquelle
R représente CF₃ , CN ou COF,
dans lequel on fait réagir un dérivé de chlorobenzène répondant à la formule (II) 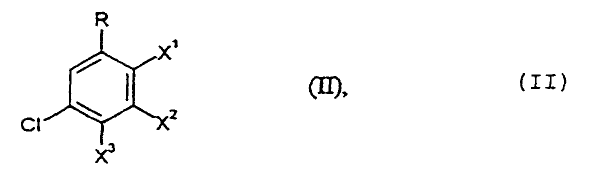 dans laquelle
R a la signification indiquée pour la formule (I) et
X¹, X² et X³ représentent, indépendamment les uns des autres, à chaque fois, F ou Cl,
avec un agent de fluoration en présence d'un solvant et d'un catalyseur à température élevée, **caractérisé en ce que** l'on effectue la réaction d'abord dans des conditions de température et de pression telles que le composé répondant à la formule (I) distille en continu par une colonne et que l'on établit ensuite des conditions de température et de pression telles que les fractions restantes du composé répondant à la formule (I) distillent en même temps que le composé 2,3,4-trifluoro-5-chloro correspondant.

2. Procédé selon la revendication 1, **caractérisé en ce que** la température de réaction se situe dans une plage de 160 à 250 °C.

3. Procédé selon les revendications 1 ou 2 , **caractérisé en ce que** l'on commence par régler la pression pour une température de réaction donnée, de telle sorte que le composé répondant à la formule (I) distille en continu.

4. Procédé selon la revendication 3, **caractérisé en ce que** l'on établit d'abord la pression de 1,3 à 6 bars.

5. Procédé selon les revendications 1 à 4, **caractérisé en ce que**, lorsqu'il ne passe pratiquement plus de composé répondant à la formule (I), on abaisse la pression de 50 à 200 mbars.

6. Procédé selon les revendications 1 à 5, **caractérisé en ce que**, dans la formule (II), X¹, X² et X³ représentent un atome de chlore.

7. Procédé selon les revendications 1 à 6, **caractérisé en ce que** l'on utilise des mélanges de composés répondant à la formule (II) dans laquelle X¹, X² et X³ représentent un atome de chlore, avec des composés répondant à la formule (II) , dans laquelle X¹, X² et X³ représentent un atome de fluor.

8. Procédé selon les revendications 1 à 7, **caractérisé en ce que** l'on utilise comme agents de fluoration des fluorures de métaux alcalins.

9. Procédé selon les revendications 1 à 8, **caractérisé en ce que** l'on utilise comme solvants des solvants aprotiques dipolaires.

10. Procédé selon les revendications 1 à 9, **caractérisé en ce que** l'on utilise comme catalyseurs des catalyseurs de transfert de phases.
